# EUROPÄISCHE PATENTSCHRIFT

(11) **EP 2 988 768 B1**
(45) Veröffentlichungstag und Bekanntmachung des Hinweises auf die Patenterteilung: **31.03.2021**
(21) Anmeldenummer: 14718432.9
(22) Anmeldetag: 18.04.2014
(51) Int. Cl.: A61K 38/19, A61K 9/00, A61P 11/00

(54) **LYOPHILISAT ENTHALTEND EIN ZYKLISCHES PEPTID DER FORMEL X1-GQRETPEGAEAKPWY-X2 ZUR BEHANDLUNG PULMONARER ÖDEME**
LYOPHILISATE COMPRISING A CYCLIC PEPTIDE OF FORMULA X1-GQRETPEGAEAKPWY-X2 FOR TREATING PULOMARY EDEMA
LYOPHILISAT COMPRENANT UN PEPTIDE CYCLIQUE DE FORMULE X1-GQRETPEGAEAKPWY-X2 POUT LE TRAITEMENT DE L'OEDEME DU POUMON

(30) Priorität: 23.04.2013 EP 13164829
(43) Veröffentlichungstag der Anmeldung: 02.03.2016
(73) Patentinhaber: APEPTICO Forschung und Entwicklung GmbH, 1150 Wien (AT)
(72) Erfinder: FISCHER, Bernhard, A-1160 Wien (AT); LUCAS, Rudolf, Martinez, GA 30907 (US)
(74) Vertreter: Vögele, Andreas
(86) Internationale Anmeldenummer: PCT/EP2014/058010
(87) Internationale Veröffentlichungsnummer: WO 2014/173842

(56) Entgegenhaltungen:
- WO-A1-2006/013183
- WO-A1-2010/099556
- WO-A1-2011/085423
- PARASTOO HAZEMI ET AL: "Essential structural features of TNF-alpha lectin-like domain derived peptides for activation of amiloride-sensitive sodium currecnt in A549 cells", JOURNAL OF MEDICINAL CHEMISTRY, AMERICAN CHEMICAL SOCIETY, Bd. 53, Nr. 22, 27. Oktober 2010 (2010-10-27), Seiten 8021-8029, XP002638326, DOI: 10.1021/JM100767P in der Anmeldung erwähnt
- VADASZ I ET AL: "The lectin-like domain of tumor necrosis factor-[alpha] improves alveolar fluid balance in injured isolated rabbit lungs", CRITICAL CARE MEDICINE 200805 US, Bd. 36, Nr. 5, Mai 2008 (2008-05), Seiten 1543-1550, XP009172075, ISSN: 0090-3493
- ELIA NADIA ET AL: "Functional identification of the alveolar edema reabsorption activity of murine tumor necrosis factor-alpha", AMERICAN JOURNAL OF RESPIRATORY AND CRITICAL CARE MEDICINE, AMERICAN LUNG ASSOCIATION, NEW YORK, NY, US, Bd. 168, Nr. 9, 1. November 2003 (2003-11-01), Seiten 1043-1050, XP002455314, ISSN: 1073-449X, DOI: 10.1164/RCCM.200206-618OC

### SEQUENCE LISTING

<110> APEPTICO Forschung und Entwicklung GmbH
<120> Pharmazeutische Zusammensetzung
<130> A 18066
<150> EP 13164829.7
   <151> 2013-04-23
<160> 8
<170> PatentIn version 3.5
<210> 1
   <211> 17
   <212> PRT
   <213> Artificial Sequence
<220>
   <223> zyklisches Peptid mit Disulfidbrücke
<220>
   <221> DISULFID
   <222> (1)..(17)
<400> 1
   Cys Gly Gln Arg Glu Thr Pro Glu Gly Ala Glu Ala Lys Pro Trp Tyr
   1               5                   10                  15
   Cys
<210> 2
   <211> 19
   <212> PRT
   <213> Artificial Sequence
<220>
   <223> zyklisches Peptid mit einer Amidbindung zwischen der an das epsilon-C-Atom des Lysinrests gebundenen Aminogruppe und der an das gamma-C-Atom des Glutaminsäurerests gebundenen Seitenketten-Carboxylgruppe
<400> 2
   Lys Ser Pro Gly Gln Arg Glu Thr Pro Glu Gly Ala Glu Ala Lys Pro
   1               5                   10                  15
   Trp Tyr Glu
<210> 3
   <211> 17
   <212> PRT
   <213> Artificial Sequence
<220>
   <223> zyklisches Peptid mit einer Amidbindung zwischen der an das epsilon-C-Atom der Seitenkette des Lysinrests gebundenen Aminogruppe und der Carboxylgruppe des Glycinrests
<400> 3
   Lys Gly Gln Arg Glu Thr Pro Glu Gly Ala Glu Ala Lys Pro Trp Tyr
   1 5 10 15
   Gly
<210> 4
   <211> 17
   <212> PRT
   <213> Artificial Sequence
<220>
   <223> zyklisches Peptid mit einer Amidbindung zwischen der an das delta-C-Atom der Seitenkette des Ornithinrests gebundenen Aminogruppe und der Carboxylgruppe des Glycinrests
<220>
   <221> MOD_RES
   <222> (1)..(1)
   <223> X = Ornithin
<400> 4
   Xaa Gly Gln Arg Glu Thr Pro Glu Gly Ala Glu Ala Lys Pro Trp Tyr
   1 5 10 15
   Gly
<210> 5
   <211> 17
   <212> PRT
   <213> Artificial Sequence
<220>
   <223> zyklisches Peptid mit einer Amidbindung zwischen der Aminogruppe des 4-Aminobuttersäurerests und der an das beta-C-Atom des Asparaginäurerests gebundenen Seitenketten-Carboxylgruppe
<220>
   <221> MOD_RES
   <222> (1)..(1)
   <223> X = 4-Aminobuttersäure
<400> 5
   Xaa Gly Gln Arg Glu Thr Pro Glu Gly Ala Glu Ala Lys Pro Trp Tyr
   1 5 10 15
   Asp
<210> 6
   <211> 17
   <212> PRT
   <213> Artificial Sequence
<220>
   <223> zyklisches Peptid mit einer Amidbindung zwischen der Aminogruppe des beta-Alaninrests und der an das gamma-C-Atom des Glutaminsäurerests gebundenen Seitenketten-Carnonylgruppe
<220>
   <221> MOD_RES
   <222> (1)..(1)
   <223> X = beta-Alanin
<400> 6
   Xaa Gly Gln Arg Glu Thr Pro Glu Gly Ala Glu Ala Lys Pro Trp Tyr
   1               5                   10                  15
   Glu
<210> 7
   <211> 17
   <212> PRT
   <213> Artificial Sequence
<220>
   <223> zyklisches Peptid mit Disulfidbrücke
<220>
   <221> DISULFID
   <222> (1)..(17)
<400> 7
   Cys Gly Gln Arg Glu Ala Pro Ala Gly Ala Ala Ala Lys Pro Trp Tyr
   1               5                   10                  15
   Cys
<210> 8
   <211> 17
   <212> PRT
   <213> Artificial Sequence
<220>
   <223> zyklisches Peptid der Formel I
<220>
   <221> MOD_RES
   <222> (1)..(1)
   <223> X = ausgewählt aus Cys; Lys-Ser-Pro; Lys; Ornithin; 4-Aminobuttersäure; beta-Alanin
<220>
   <221> MOD_RES
   <222> (17)..(17)
   <223> X = ausgewählt aus Cys; Asp; Gly, Glu
<400> 8
   Xaa Gly Gln Arg Glu Thr Pro Glu Gly Ala Glu Ala Lys Pro Trp Tyr
   1               5                   10                  15
   Xaa

## Beschreibung

Die vorliegende Erfindung betrifft ein zyklisches Peptid der Formel X₁-GQRETPEGAEAKPWY-X₂ zur Verwendung als Arzneimittel.

Die Wirkung von Arzneistoffen ist sehr wesentlich davon abhängig, wie diese Wirkstoffe in den Körper bzw. das zu behandelnde Organ gelangen.

Die übliche Art und Weise der Verabreichung eines Medikamentes ist die parenterale Verabreichung. Hier wird ein Medikament durch die Haut injiziert, z.B. direkt in die Blutbahn oder in einen Muskel oder direkt unter die Haut.

Eine andere geläufige Form der Medikamenteinnahme besteht in der oralen Gabe in den Magen- und Darmtrakt. Die in den Medikamenten enthaltenen Wirkstoffe werden im Magen- und Darmtrakt freigesetzt und gelangen dann mittels Resorption in den Körper, das Blut und die Organe.

Medikamente können auch durch die Haut und Schleimhaut verabreicht werden.

Als Alternative zur Injektion wurden Verfahren zur oralen Inhalation von Medikamenten vorgeschlagen. Hierbei werden Medikamente sowohl als Pulver oder in Tröpfchenform in den Mund- und Rachenraum abgegeben, welche dann mit der Atemluft möglicherweise in den Lungenraum gelangen. Von dort werden diese Medikamente über das Lungengewebe an das Blut abgegeben, und so systemisch dem Körper zugeführt.

Die orale Inhalation von Medikamenten stellt jedoch heute noch immer ein wesentliches technisches Problem dar. Insbesondere hochmolekulare Wirkstoffe, wie zum Beispiel Proteine, lassen sich mittels oraler Inhalation nur sehr begrenzt applizieren. Die Proteine als Wirkstoffe werden durch den Vorgang des Zerstäubens oder Vernebeln physikalisch durch Hitze und Drücke geschädigt und inaktiviert. Zur Inhalation vorgesehene Protein-Wirkstoffe sind sehr instabil, sowohl während deren Lagerung vor- und während der oralen Inhalation. Zudem können solche Peptid-Wirkstoffe durch physiologische Abbauprozesse schon im Luftraum der Lunge abgebaut werden.

Um dem entgegenzuwirken wurden in solchen Präparationen zur Inhalation von Proteinen unterschiedlichste pharmazeutische Formulierungen entwickelt. So können solche Zubereitungen zur Inhalation von Proteinen Salze, wie Calcium oder Natriumsalze, Stabilisatoren und Tenside, spezifische Puffermischungen, Lipid-Beimischungen und anderes zur Erzielung von gewünschten Lösungseigenschaften, zur Gewährleistung der Stabilität, zum Schutz der Aktivität des Proteins, enthalten. Andere Zusatzstoffe während der Herstellung von Protein-Arzneimitteln beinhalten zum Beispiel Albumin, osmolische Reagenzien, Antioxidanzien, chemische Stoffe zur Vermeidung von Aggregation und Präzipitation, Liposomen, Gelantine, Alginate, Zucker, etc..

Solcherart hergestellte und pharmazeutisch zubereitete Peptid- und Protein-Wirkstoffe zur Inhalation gelangen dann beabsichtig oder unbeabsichtigt durch das Lungengewebe in das Blut und können dort nachgewiesen werden.

Insgesamt gibt es derzeit nur zwei international zugelassene Protein/Peptid basierte Medikamente zur Inhalation, nämlich Pulmozyme® (Desoxyribonuklease) und Exubera® (Insulin). Zur Gewährleistung der Aktivität und Stabilität der Präparate enthalten diese z.B. Salze (Natriumchlorid, Calziumchlorid, bzw. Natriumcitrat, Manitol, Glyzerin, Natriumhydroxid).

Ein technisch üblicher Weg zur Herstellung von Protein/Peptid-Formulierungen zur Humanmedizinischen Anwendung ist die Lyophilisation zum Entzug von Wasser (z.B. J Pharm Sci. 2009 Sep;98(9):2886-908). Bei diesen State-of-the-Art Prozessen werden jedoch Zusätze, wie z.B. Disaccharide, zugegeben, um die Stabilität und Aktivität der Peptide zu gewährleisten (Allison SD et al, Arch Biochem Biophys. 1999 May 15;365(2):289-98). Darüber hinaus hat es sich bewährt, an Peptide und Proteine Polyethylenglycol (PEG) anzubinden, um nach dem Wasserentzug bei Lyophilisation die Aktivität und Stabilität zu gewährleisten (Roberts et al, Adv Drug Deliv Rev. 2002 Jun 17;54(4):459-76 2002, Morris et al, Antimicrob Agents Chemother. 2012 Jun;56(6):3298-308. doi: 10.1128/AAC.06335-11), sowie um die Degradation durch proteolytische Prozesse zu verhindern (Lee et al, Regul Pept. 2009 Jan 8;152(1-3):101-7; Baginski et al, Pharm Res. 2012 Jun;29(6):1425-34), bzw. um die Molekülmasse günstig zu beeinflussen (Veronese & Pasut, Drug Discov Today. 2005 Nov 1;10(21): 1451-8; Patton & Byron, Nat Rev Drug Discov. 2007 Jan;6(1):67-74).

Es wurde nun für bestimmte Peptide, die einen positiven Einfluß auf die Funktion der Lunge haben, überraschenderweise eine Anwendungsform ohne Zusatzstzoffe gefunden die sich als überaus geeignet herausgestellt hat.

In einem Aspekt stellt die vorliegende Erfindung ein zyklisches Peptid der Formel

X₁-GQRETPEGAEAKPWY-X₂ I

zur Verfügung, worin
X₁ eine Aminsäure(sequenz) umfasst, mit 1 bis 4 Mitgliedern, umfassend natürliche oder unnatürlicheAminosäuren, und
X₂ eine natürliche Aminsäure umfasst, und worin
X₁ die N-terminale Aminosäure links in Position 1, und X₂ die C-terminale Aminosäure in der letzten, rechten Position enthält,
zur Verwendung als Arzneimittel zur Behandlung von pulmonaren Ödemen in der Form eines wässrigen Aerosols zur Inhalation ohne Zusatzstoffe und Stabilisatoren, erhältlich aus einem Lyophilisat des zyklischen Peptids der Formel I ohne Zusatzstoffe und/oder Stabilisatoren.

Ein Lyophilisat wird hier auch als "Lyophilisat der vorliegenden (nach, gemäß vorliegender) Erfindung" bezeichnet. Ein Peptid in einem Lyophilisat gemäß vorliegender Erfindung wird hier auch als "Peptid der vorliegenden (nach, gemäß vorliegender) Erfindung" bezeichnet.

In einem Lyophilisat der vorliegenden Erfindung kann ein oder können mehrere zyklische Peptide der Formel I vorliegen, wobei bevorzugt nur ein Peptid der Formel I vorliegt.

Eine zyklische Verbindung der Formel I ist die zyklisiserteVerbindung mit der Aminosäuresequenz

X₁-Gly-Gln-Arg-Glu-Thr-Pro-Glu-Gly-Ala-Glu-Ala-Lys-Pro-Trp-Tyr-X₂ I,

worin X₁ und X₂ wie oben definiert sind..

Der Ring in einer Verbindung der Formel I kann durch Bindung zwischen zwei geeigneten Substituenten in zwei der Aminosäurereste der Verbindung der Formel I ausgebildet sein, z.B. durch eine Amidbindung, oder eine Disulfidbrücke, wobei der Ring vorzugsweise mindestens 15, mehr bevorzugt mindestens 17, bis zu 19 oder 20, z.B. 17 bis19 Aminosäurereste, die in einer Verbindung der Formel I vorliegen, als Ringglieder umfasst.

Vorzugsweise ist die Ringbildung ausgebildet durch Bindung zwischen einem geeigneten Substiuenten in einer Aminosäure in X₁, vorzugsweise in der Aminosäure in Position 1 in X₁, mit einem geeigneten Substituenten in X₂.

Natürliche Aminosäuren, die in einer Aminosäuresequenz der Formel I der vorliegenden Erfindung angewendet werden können, sind bekannt und umfassen z.B. G (Gly), A (Ala), V (Val), L (Leu), I (Ile), M (Met), P (Pro), F (Phe), W (Trp), S (Ser), T (Thr), N (Asn), Q (Gln), C (Cys), U (Sec), Y (Tyr), D (Asp), E (Glu), H (His), K (Lys), R (Arg).

Unnatürliche Aminosäuren, die in einer Aminosäuresequenz der Formel I der vorliegenden Erfindung angewendet werden können, umfassen
(i) Aminosäuren, die die prinzipielle chemische Struktur natürlicher Aminosäuren aufweisen, die aber anders sind, als alpha Aminosäuren,
(ii) natürliche Aminosäuren in derD-Form, nämlich anders, als in der natürlichen L-Form, das heisst natürliche Aminosäuren, worin die Alkylgruppe am C-Atom in Position 2 nicht in der L-Konfiguration, sondern in der D-Konfiguration vorliegt,
(iii) unnatürliche Aminosäuren, z.B. andere als inter (i) und (ii) oben definiert, umfassen von 2 bis 12, wie von 2 bis 6 Kohlenstoffatome, zumindestens eine Aminogruppe, z.B. eine, oder zwei, und zumindestens eine Carboxygruppe, z.B. eine, oder zwei, , gegebenenfalls neben Substituenten, die auch in natürlichen Aminpsäuren vorhanden sind, z-B. OH, -CONH₂,-NH-C(=NH₂)NH₂, SH, (C₁₋₄)Alkyl-S-, Phenyl, Heterocyclyl, z.B. umfassend 5 oder 6 Ring Mitglieder und umfassend zumindestens ein Heteroatom, z.B. ein oder zwei, ausgewählt von N, O, S, bevorzugt N, gegebenenfalls annelliert mit einem weiteren Ring, wie Phenyl, z.B. umfassend Prolinyl, Indolyl, Imidazolyl;

Unnatürliche Aminosäuren, der Formel I der vorliegenden Erfindung umfassen Ortithin, 4-Aminobuttersäure, β-Alanin.

In einem weiteren Aspekt ist die Verbindung der Formel I ausgewählt aus der Gruppe der Peptide der Aminosäuresequenzen
- Aminosäuresequenz SEQ ID NO: 1
   *Cyclo*(CGQRETPEGAEAKPWYC)
   worin zwischen den beiden terminalen Cysteinresten eine Disulfidbrücke ausgebildet ist;
- Aminosäuresequenz SEQ ID NO:2
   *Cyclo*(KSPGQRETPEGAEAKPWYE)
   worin eine Amidbindung ausgebildet ist zwischen der Aminogruppe, die an das ε-Kohlenstoffatom des N-terminalen Lysinrestes gebunden ist und der Seitenketten-Carboxylgruppe, die an das γ-Kohlenstoffatem des C-terminalen Glutaminsäurerestes gebunden ist;
- Aminosäuresequenz SEQ ID NO:3
   *Cyclo*(KGQRETPEGAEAKPWYG)
   worin eine Amidbindung ausgebildet ist zwischen der Aminogruppe, die an das ε-Kohlenstoffatom der Seitenkette des N-terminalen Lysinrestes gebunden ist mit der Carboxylgruppe des C-terminalen Glycinrestes;
- Aminosäuresequenz SEQ ID NO:4
   *Cyclo*(Ornithin-GQRETPEGAEAKPWYG)
   worin eine Amidbindung ausgebildet ist zwischen der Aminogruppe, die an das δ-Kohlenstoffatom der Seitenkette des N-terminalen Ornithinrestes gebunden ist und der Carboxylgruppe des C-terminalen Glycinrestes;
- Aminosäuresequenz SEQ ID NO:5
   Cyclo(4-Aminobuttersäure-GQRETPEGAEAKPWYD)
   worin eine Amidbindung ausgebildet ist zwischen der Aminogruppe des N-terminalen 4-Aminobuttersäurerestes und und der Seitenketten-Carboxylgruppe, die an das β-Kohlenstoffatom des C-terminalen Aspagraginsäurerestes gebunden ist;
   und
- Aminosäuresequenz SEQ ID NO:6
   Cyclo(β-alanine-GQRETPEGAEAKPWYE)
   worin eine Amidbindung ausgebildet ist zwischen der Aminogruppe des N-terminalen β-Alaninrestes (3-Aminopropansäurerest) und der Seitenketten-Carboxylgruppe, die an das γ-Kohlenstoffatom des C-terminalen Glutaminsäurerestes gebunden ist.

In den Verbindungen der Formel I, wie den Verbindungen mit den Aminosäuresequenzen SEQ ID NO:1, SEQ ID NO:2, SEQ ID NO:3, SEQ ID NO:4, SEQ ID NO:5 oder SEQ ID NO:6 sind die Aminosäureren normal peptidisch verknüpft, mit Ausnahme jener Bindung, die zur Ringbildung führt.

Eine zyklische Verbindung der Formel I wird hier auch als "zyklische Verbindung(en) der vorliegenden (nach, gemäß vorliegender) Erfindung" bezeichnet und umfasst eine zyklische Verbindung der Formel I in jeglicher Form, z.B. in freier Form und in der Form eines Salzes. In biologischer Umgebung liegt normalerweise eine Verbindung der Formel I in der Form eines Salzes vor.

In einem weiteren Aspekt liegt eine Verbindung der Formel I in einem Lyophilisat der vorliegenden Erfindung in der Form eines Salzes vor.

Solche Salze schließen vorzugsweise pharmazeutisch annehmbare Salze ein, obwohl pharmazeutisch nicht annehmbare Salze mit umfasst sind, z.B. zum Zwecke der Herstellung/Isolations/Reinigung.

In biologischer Umgebung ist ein Salz einer zyklischen Verbindung der Formel I normalerweise ein Hydrochlorid.

Eine zyklische Verbindung der Formel I der vorliegenden Erfindung in freier Form kann in eine zyklische Verbindung der Formel I in der Form eines Salzes umgewandelt werden und vice versa.

Eine zyklische Verbindung der Formel I der vorliegenden Erfindung kann in der Form von Isomeren und Isomerengemischen vorliegen; z.B. optischen Isomeren. Eine zyklische Verbindung der Formel I kann z.B. asymmetrische Kohlenstoffatome enthalten und kann daher in der Form von Enantiomeren, Diastereoisomeren und Mischungen davon, z.B. Racematen vorliegen. Eine zyklische Verbindung der Formel I kann in der (R)-, (S)- oder (R,S)-Konfiguration vorzugsweise in der (R)- oder (S)-Konfiguration betreffend einzelne Substiuenten an asymmetrischen Kohlenstoffatomen vorliegen. Isomerenmischungen können in geeigneter Weise getrennt werden, z.B. gemäß, z.B. analog, einer konventionellen Methode, um reine Isomeren zu erhalten. Die vorliegende Erfindung umfasst eine zyklische Verbindung der Formel I der vorliegenden Erfindung in jeglicher Isomerenform und in in jeglicher Form von Isomerenmischungen. Im Falle natürlicher Aminosäuren ist die Konfiguration von Substituenten normalerweise so, wie in natürlichen Aminosäuren.

Eine zyklische Verbindung der Formel I der vorliegenden Erfindung kann in geeigneter Weise hergestellt warden, z.B. gemäß, beispielsweise analog, zu konventionellen Methoden, oder wie hier beschrieben, z.B. durch Festphasen Peptidsynthese, gegebenenfalls gemäß der Schützungsstrategie mit Fluorenylmethoxycarbonyl / t-Butyl an 2-Chlorotritylchloridharz unter Verwendung geeigneter Kopplungsvermittler, wie Diisopropylcarbodiimid und/oder N-Hydroxybenzotriazol und geeignetem Lösungsmittel, z.B. N,N-Dimethylformamid. Geschützte Aminosäuren können nacheinander an die Peptidkette angekoppelt werden, wobei mit der C-terminalen Aminosäure begonnen werden wird. Entschützung von Fluorenylmethoxycarbonyl-geschützten Gruppen kann mit Hilfe einer Base erfolgen, z.B. Piperidin, wie 20% Piperidin in geeignetem Lösungsmittel, wie N-N-Dimethyl formamid. Die Abspaltung des fertigen, gegebenenfalls noch (teil)geschützten Peptides vom Träger(Harz) kann in geeigneter Weise erfolgen, z.B. mit Hilfe einer Säure, wie Essigsäure in geeignetem Lösungsmittel, z.B. in einem halogenierten Kohlenwasserstoff, wie CH₂Cl₂, z.B. in einer Mischung aus Essigsäure und CH₂Cl₂.in einem Verhältnis von 1:1.

Im Falle Cystein-hältiger Peptide kann nach Abspaltung vom Träger(Harz) Entschützung erfolgen, falls nötig, z.B. mit einer starken Säure, wie Trifluoroessigsäure (TFA), z.B. 95% TFA / 5% H₂O. Die Zyklisierung zum Erhalt einer Disulfidbindung kann durch Oxidation der terminalen Cysteinreste erfolgen, z.B. durch Behandlung des rohen, linearen Peptids mit Luft, z.B. bei einem pH von 8.5 für 90 Stunden. Eine Reinigung des rohen Peptids, das erhalten wird, kann erfolgen, etwa mittels Chromatographie, z.B. Reverse Phase Medium Pressure Liquid Chromatography (RP-MPLC) über eine geeignete Säule, wie eine RP-C18-Silicagel Säule, wobei günstigerweise eine Eluentgradient angewendet werden kann, wie ein 5% - 40% Acetonitril-Wasser-Gradient. Ein Trifluoroessigsäure Gegenion kann ersetzt werden, z.B. durch Acetat, beispielsweise mit Hilfe einer Säule, wie einer Lewatit MP64 Säule (Acetat Form). Nach einem letzten Waschen mit Wasser kann das gereinigte Peptid als Acetatsalz lyophilisiert und in der Form einer leicht gefärbten Verbindung, z.B. in der Form eines weißen Pulvers, erhalten werden.

Im Falle Cystein-freier Peptide kann der Zyklisierungsschritt in geeigneter Weise ausgeführt werden, z.B. noch an dem teilgeschützten linearem Peptid nach der Abspaltung vom Träger(Harz). Nach selektiver Zyklisierung des Cysteinfreien Peptids, kann eine Seitenkettenentschützung in TFA erfolgen, falls nötig. Ein Reinigungsschritt kann ausgeführt werden, z.B. mittels Chromatographie, z.B. durch präparative RP-MPLC. Im Peptid, das dabei erhalten wird kann das Trifluoroessigsäureion durch das Acetation ersetzt werden, z.B. so, wie oben beschrieben. Lyophilisierung der Acetatform des Peptids, das dabei erhalten wiord, kann ebenfalls erfolgen, z.B. so, wie für die Cystein enthaltenden Peptide.

Das Molekulargewicht erhaltener Peptide kann durch Electrospray-Ionisations-Massenspektrometrie oder MALDI-TOF-MS bestätigt werden. Die Reinheit kann z.B. durch analytische H(igh)P(erformance)L(iquid)C(hromatography) bestimmt werden,
Zyklische Verbindungen der Formel I der vorliegenden Erfindung beinhalten Verbindungen mit den Aminosäuresequenzen SEQ ID NO:1, SEQ ID NO:2, SEQ ID NO:3, SEQ ID NO:4, SEQ ID NO:5 oder SEQ ID NO:6. Solche Verbindungen sind z.B. aus Hazemi P., Tzotzos, S. Fischer B., Andavan, G.S.B., Fischer H., Pietschmann H, Lucas, R. und Lemmens-Gruber, R. in J Med Chem. 2010 November 25, 53(22): 8021-8029, "Essential structural features of TNF-α lectin-like domain derived peptides for activation of amiloride-sensitive sodium current in A549 cells" als den "Amilorid-sensitiven epithelialen Natriumkanal (ENaC)"-aktivierend und somit als nützlich zur Behandlung von Krankheiten in der Lunge bekannt. Durch die Aktivierung des Amilorid-sensitiven Natrium Ionen Kanal (ENaC) kommt es zum Transport von Natrium-Ionen durch das Lungengewebe. Es bildet sich ein osmotischer Gradient aus, welcher zum passiven Wassertransport führt. Wird dieses Model auf die Lunge übertragen, kann die Aktivierung des Amilorid-sensitiven Natrium Ionen Kanals beispielsweise zur Verringerung von Wasseransammlung in der Lunge verwendet werden, z.B. im Falle pulmonarer Oedema.

Dabei hat sich im Zuge der Entwicklung der vorliegenden Erfindung herausgestellt, dass ein aktiver oder passiver Transport der zyklischen Peptide der Formel I der vorliegenden Erfindung, in der X₁ und X₂ wie oben definiert sind, beispielsweise mit der Aminosäuresequenz SEQ ID NO:1 bis SEQ ID NO:6, durch das Lungengewebe in das Blut nicht wünschenswert ist und nicht stattfinden soll, da es wesentlich zur physiologischen Wirksamkeit der zyklischen Peptide der Amionosäuresequenzen SEQ ID 1 bis SEQ ID 6 beiträgt, wenn diese mittels oraler Inhalation in den Luftraum der Lunge gelangen, sodass sie sich auf die Oberfläche des Lungengewebes abscheiden und dort den apikal orientierten Amilorid-sensitiven Natrium Ionen Kanal aktivieren können.

Es konnte überraschenderweise gezeigt werden (Beispiel 10), dass nach Inhalation eines Aerosols, das aus einem Lyophilisat gemäß vorliegender Erfindung durch Zugabe von Wasser hergestellt ist, ein Peptid gemäß vorliegender Erfindung nicht im Blut nachweisbar ist.
Inhalative Präparate wurden und werden aber normalerweise entwickelt, damit diese nach der Inhalation über das Lungengewebe in die Blutbahn übertreten, und somit eine parenteral Injektion vermieden werden kann. Ziel bisheriger Inhalationsmedikamente ist nämlich die systemische Wirkung. Bei der Verteilung eines Wirkstoffes im Blut gelangt das Molekül in jedes Gewebe und Organ. Ein wesentliche Nachteil von systemischer Applikation ist allerdings das große Spektrum an Toxizität und Nebenwirkungen an Organen und Geweben des Körpers, welche nicht mit der zu behandelten Krankheit in Zusammenhang stehen.

Bei Anwendung eines wässrigen Aerosols, das aus einem Lyophilisat gemäß vorliegender Erfindung hergestellt ist, gelangen im Gegensatz dazu die Peptide in die Grenzschicht zwischen Luft und Lungenepithel, wo sie einen Natrium-Ionenkanal aktivieren. Die Peptide gemäß vorliegender Erfindung wirken also "lokal" auf das Epithelgewebe der Lunge und nicht systemisch. Ein Durchtritt der Peptide durch das Lungengewebe ist nicht gewünscht und findet auch nicht statt.

Im Unterschied zu den aus der Literatur bekannten Peptid-Formulierungen, ist es durch das wässrige Aerosol, das aus einem Lyophilisat gemäß vorliegender Erfindung hergestellt wurde und das keine Zusatzstoffe enthält, gelungen, eine Diffusion des inhalierten Peptides durch die Lunge ins Blut zu verhindern. Dadurch ergibt sich der äußerst positive Effekt, dass mögliche systemisch-toxische Eigenschaften des Peptides, auch auf andere Organe, als auf die Lunge, zu verhindern Sonst übliche systemische Nebenwirkungen können somit ausgeschlossen werden, was ein großer medizinischer Nutzen darstellt. Durch die lokale Gabe auf die innere Lungenoberfläche und ohne Diffusion ins Blut, wird, im Vergleich zur systemischen Applikation dazu noch deutlich weniger Wirkstoff benötigt.

Es hat sich weiterhin überraschend herausgestellt, dass bei einem Lyophilisat gemäß vorliegender Erfindung, das zyklischen Peptide der Formel I, insbesondere die zyklischen Peptide der Aminosäuresequenzen SEQ ID NO: 1 bis SEQ ID NO:6 ohne Zusatzstoffe enthält, über Monate und Jahre eine chemische oder biologische Instabilität praktisch ausgeschlossen werden kann. Es hat sich nämlich herausgestellt, dass die chemische Struktur und die biologische Aktivität der zyklischen Peptide der Formel I der vorliegenden Erfindung, wie jener mit den Aminosäuresequenzen SEQ ID NO:1 bis SEQ ID NO:6 nicht geschädigt wird, überraschenderweise auch ohne Zusatz allgemein üblicher Zusatzstoffe und/oder Stabilisatoren.

Bei der Anwendung der zyklischen Peptide der Formel I der vorliegenden Erfindung, insbesondere der Aminosäuresequenzen SEQ ID NO:1 bis SEQ ID NO:6, zur Inhalation hat sich herausgestellt, dass die zyklischen Peptide so beschaffen sind, dass diese auch in aufgelöster Form, nämlich in wässriger Lösung, auch ohne Zusatzstoffe, längere Zeit stabil sind, zum Beispiel im Vorratsbehälter eines Gerätes zum Vesprühen (Vernebler). Außerdem hat sich gezeigt, dass die zyklischen Peptide der Formel I der vorliegenden Erfindung, insbesondere der Aminosäuresequenzen SEQ ID NO:1 bis SEQ ID NO:6, gemäß vorliegender Erdindung so zubereitet werden können, dass bei deren Überführung in eine inhalierbare Form die zyklischen Proteine SEQ ID 1 bis SEQ ID 6 nicht geschädigt werden und somit die volle Aktivität beibehalten, überraschenderweise auch ohne Zusatz allgemein üblicher Zusatzstoffe und/oder Stabilisatoren.

Zur Herstellung eine Lösung eines, z.B. mindestens eines, der Peptide der Formel I der vorliegenden Erfindung, insbesondere eines mit der Aminosäuresequenz SEQ ID NO:1 bis SEQ ID NO:6 in der Form, die als Aerosol zur Versprühung geeignet ist, können diese Peptide in Wasser aufgelöst werden, und die erhaltene Lösung, beispielsweise ohne weitere Zusätze, wie z.B. übliche Zusatzstoffe und/oder Stabilisatoren, lyophilisert werden, wobei ein Pulver erhalten wird. Vor dem Lyophilisieren kann die Lösung gegebenenfalls filtriert werden, um Trübstoffe zu entfernen.

In einem weiteren Aspekt stellt die vorliegende Erfindung die Verwendung eines Lyophilisats gemäß vorliegender Erfindung, zur Herstellung eines Aerosols, das zur Versprühung geeignet ist, ohne Zusatzstoffe und/oder Stabilisatoren, zur Verfügung.

Die Verwendung eines Lyophilisats gemäß vorliegender Erfindung wird hier auch als "Verwendung gemäß (nach) vorliegender Erfindung" bezeichnet.

Es wurde gefunden, dass Peptide der Formel I, insbesondere jene mit der Aminosäuresequenz SEQ ID NO:1 bis SEQ ID NO:6, in derart lyophilisierter Form über mindestens 24 Monate in gekühlter Umgebung und bei Raumtemperatur über mindestens 6 Monate stabil waren, sogar ohne den Zusatz von sonst üblichen Zusatzstoffen und/oder Stabilisatoren.

Ein Lyophilisat gemäß vorliegender Erfindung kann zur Anwendung in Wasser zum Erhalt eines Aeroslos ohne Zusätze aufgelöst werden, z.B. zur direkten Herstellung einer Aerosols oder zur Aufbewahrung als Lösung, z.B. in einem Vorratsbehälter.

In einem weiteren Aspekt stellt die vorliegende Erfindung die Verwendung eines Lyophilisats zur Verfügung, worin ein Peptid der Formel I, in der X₁ und X₂ wie oben definiert sind in wässriger Lösung, ohne Zusatzstoffe und/oder Stabilisatoren, vorliegt.

Es hat sich überraschenderweise und vorteilhaft herausgestellt, dass die derart aufgelösten zyklischen Peptide der Formel I, in der X₁ und X₂ wie oben definiert sind, insbesondere mit den Aminosäuresequenzen SEQ ID NO:1 bis SEQ ID NO:6, über mindestens 7 Tage in Lösung stabil waren, das heißt, dass die biologische Wirksamkeit der zyklischen Proteine durch die Überführung in den inhalierbaren Zustand nicht verringert wurde, sogar ohne den Zusatz von sonst üblichen Zusatzstoffen und/oder Stabilisatoren.

Bei der Anwendung der zyklischen Peptide gemäß vorliegender Erfindung, insbesondere der Aminosäuresequenzen SEQ ID NO:1 bis SEQ ID NO:6, hat sich herausgestellt, dass eine Lösung der zyklischen Peptide so beschaffen ist, dass diese in ein Aerosol umgewandelt werden können, zum Beispiel mit Hilfe eines Verneblers, durch den die zyklischen Peptide versprüht werden. Dabei hat sich gezeigt, dass Aerosolpartikel mit Partikeldurchmessern kleiner gleich 5 µm erhalten wurden.

Es hat sich weiter herausgestellt, dass ein Aerosol, welches überwiegend Tröpfchen mit Durchmesser von ≤ 5 µm enthält, zur Anwendung besonders geeignet ist, da dadurch das Aerosol auch in den Luftraum der Lunge gelangt. Die Untergrenze der Tröpfchengröße ergibt sich einfach durch die Machbarkeit der Tröpfchengröße.

In einem weiteren Aspekt stellt die vorliegende Erfindung die Verwendung eines Lyophilisats zur Herstellung eines Aerosols, in der die Partikelgröße einen Durchmesser von ≤ 5 µm aufweist zur Verfügung.

Die vorliegende Erfindung beschreibt die Verwendung eines Lyophilisats das dadurch gekennzeichnet ist, dass das aus dem Lyophilisat hergestellte Aerosol zur Verbesserung/Konditionierung der Lunkenfunktion in der Form einer Inhalation verwendet wird.

Die vorliegende Erfindung stellt die Verwendung eines aus dem Lyophilisat gemäß vorliegender Erfindung hergestellte Aerosols, ohne Zusatzstoffe, zur Behandlung von pulmonaren Ödemen, zur Verfügung, und in einem weiteren Aspekt ein Verfahren zur Behandlung von pulmonaren Ödemen, das dadurch gekennzeichnet ist, dass einem Patienten ein Aerosol ohne Zusatzstoffe gemäß vorliegender Erfindung in der Form einer Inhalation verabreicht wird; und, in einem weiteren Aspekt, ein Lyophilisat gemäß vorliegender Erfindung zur Verwendung zur Verbesserung/Konditionierung der Lunkenfunktion, bzw. zur Inhalation zur Verbesserung/Konditionierung der Lunkenfunktion.

Die wirksame Dosis hängt von verschiedenen Faktoren ab, z.B. von der chemischen Natur und der Pharamkokinetik einer zyklischen Verbindung der Formel I, dem individuellen Verbraucher, z.B. dessen Körpergewicht, dem Alter und der individuellen Kondition des Patienten der Natur und Schwere der Krankheit. Im allgemeinen kann man für eine erfolgreiche Behandlung in größeren Säugetieren, wie z.B. Menschen, davon ausgehen, dass eine (tägliche) Dosis von (ca.) 0.1 mg/kg Körpergewicht bis etwa (ca.) 200 mg/kg, beispielswiese 1 mg/kg Körpergewicht bis 100 mg/kg Körpergewicht, verabreicht beispielsweise in mehreren, z.B. bis zu 4, Teildosen, erfolgreiche Ergebnisse liefert. Kinder erhalten üblicherweise die Hälfte der Dosis eines Erwachsenen.

Untersuchungen haben ferner gezeigt, dass sich durch Behandlung einer Spenderlunge ex vivo, nämlich vor der Transplantation in einen Patienten, mit einem zyklischen Peptid der Formel I, in der X₁ und X₂ wie oben definiert sind, insbesondere mit den Aminosäuresequenzen SEQ ID NO:1 bis SEQ ID NO:6, überraschenderweise Lungenfunktionen verbessert/konditioniert werden können. Dabei hat sich gezeigt, dass eine außerordentliche Verbesserung dadurch erreicht werden kann, wenn die zu transplantierende Lunge vor der Transplantation mit einem Aersol aus einem Lyophilisat gemäß vorliegender Erfindung, insbesondere ohne Zusatzstoffe, behandelt wird.

Beschrieben wird auch ein extracorporales Verfahren zur Verbesserung/Konditionierung von Lungenfunktionen, das dadurch gekennzeichnet ist, dass eine Spenderlunge ex vivo mit einem wässrigen Aerosol, das insbesondere ohne Zusatzstoffe und/oder Stabilisatoren, aus einem Lyophilisat gemäß vorliegender Erfindung hergestellt ist, besprüht wird.

Die Behandlung der Lunge kann auch (noch) nach erfolgter Transplantation im Empfänger erfolgen.

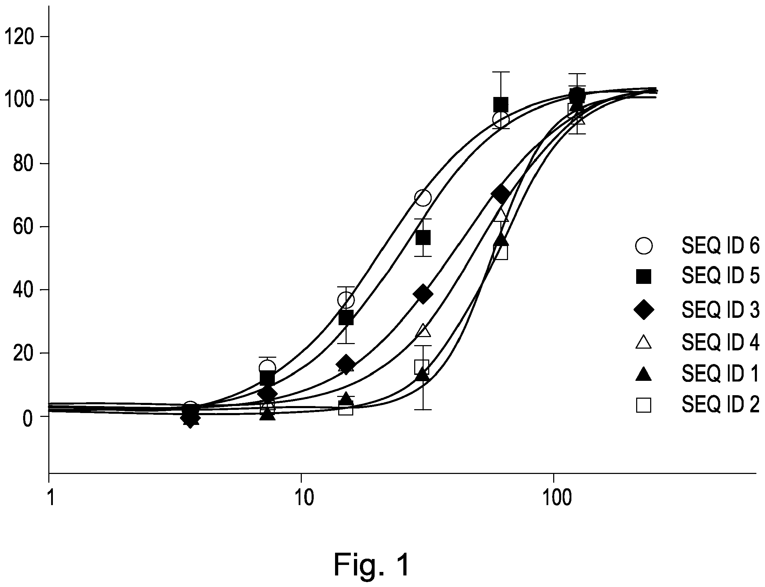

Dabei wurden Resultate erhalten, wie in der Fig. 1 gezeigt. Die Konzentration eines zyklischen Peptides der Fromel I gemäß vorliegender Erfindung im Aerosol betrug dabei von 5 nM bis ca. 150 nM.

### Beschreibung der Abbildungen

Fig. 1 zeigt die Aktivität der zyklischen Peptide mit der Aminosäuresequenz SEQ ID NO:1 bis SEQ ID NO:6 in Abhängigkeit von der Konzentration. Auf der x-Achse ist die Konzentration in nM (logarithmische Skala) der zyklischen Proteine SEQ ID NO:1 bis SEQ ID NO:6 angegeben, auf der y-Achse der Natrium Ionenstrom in %.
Fig. 2 zeigt Resultate inhalativer Anwendung eines zyklischen Peptids mit der Aminosäuresequenz SEQ ID NO:1 während einer Lungenperfusion außerhalb des Körpers (extra-corporal, ex vivo), die eine Lungentransplantation simuliert.
   In der Fig. 2A sind auf der x-Achse Zeitpunkte T1 bis T4 angegeben, an denen die Messungen nach inhalativer Anwendung des Peptids mit der Aminosäuresequenz SEQ ID NO:1 (einmal pro Stunde) erfolgten. Auf der y-Achse ist die Komplianz (Compliance) aufgetragen und in der Fig. 2B auf der x-Achse wiederum die Zeitpunkte T1 bis T4 und auf der y-Achse die arterio-venöse pO2 Differenz ΔpO2. Als Kontrolle wurde Wasser eingespritzt (WFI). Gezeigt sind die Resultate von 8 Experimenten pro Gruppe.
Fig. 3 zeigt den Lyophilisationszyklus der Peptide mit den Aminosäuresequenzen SEQ ID NO:1 bis SEQ ID NO:7, dargestellt als Fachtemperatur (ausgezogene Linie) und Produkttemperatur (gestrichelte Linie) in °C (y-Achse) mit der Zeit in Minuten (x-Achse).
Fig. 4 zeigt einen schematischer Versuchsaufbau zur Kondensierung von Aerosol in einer Kühlfalle, worin
   1 einen Polystyrolbehälter, gefüllt mit Eis und Salz,
   2 ein Röhrchen mit dem gelösten Peptid (Kontrollsubstanz),
   3 einen Vernebler,
   4 eine Vorratskammer, und
   5 ein Kontrollmodul
   bedeuten.
Fig. 5 zeigt die durchschnittliche Partikelgrößenverteilung des Aerosols des zyklischen Peptids mit der Aminosäuresequenz SEQ ID NO:1, generiert mit 2 verschiedenen Verneblern (Typ A und Typ B). Die Messung erfolgte mittels Laserdiffraktion (Flußrate: 15 L/min). Fehlerindikatoren = SD. Die Linien zeigen den jeweiligen Anteil an Partikel mit einem Durchmesser ≤ 5 µm im Aerosol. Auf der x-Achse ist die Tröpfchengröße in µm auf der y-Achse ist die kumulative Menge in % aufgetragen.
Fig. 6 zeigt Verteilung des Aerosols des zyklischen Peptids mit der Aminosäuresequenz SEQ ID NO:1 im Atemsimulator am jeweiligen Ort:
   1 = Inhalationsfilter
   2 = Exhalationsfilter
   3 = Filterverbindungsstück
   4 = Vernebler Y-Stück (inklusive Einwegeventil)
   5 = Rückstand im Vernebler),
   wobei 2 Vernebler, einer vom Typ A und einer vom Typ B eingesetzt wurden.

### Beispiel 1

### Peptidsynthese

Die Peptide wurden unter Einhaltung folgender Schritte hergestellt: Sequenzielle Kopplung der Aminosäuren; Selektive Abspaltung von der Festphase; Reinigung und Lyophilisierung, Selektive Zyklisierung; Abspaltung der Schutzgruppen; Reinigung und Lyophilisierung; Analytische Untersuchung.

Alle zyklischen Peptide der vorliegenden Erfindung, umfassend die Peptide mit den Aminosäresequenzen SEQ ID NO:1 bis SEQ ID NO:6, sowie das Peptid mit der Aminosäresequenz SEQ ID NO:7 wurden mittels der Fluorenylmethoxycarbonyl (Fmoc) / t-Butyl Schutzgruppenstrategie in Form einer Festphasensynthese an einem Träger (2-Chlorotritylchlorid-Harz) vollautomatisch hergestellt. Diisopropylcarbodiimid and N-Hydroxybenzotriazole wurden als Kopplungsvermittler verwendet. Alle Kopplungsreaktionen wurden in N,N-Dimethylformamid als Lösungsmittel ausgeführt. Die geschützten Aminosäuren wurden dazu sequenziell an die jeweils C-terminale Aminosäure, die als Startmaterial verwendet wurde, gekoppelt. Die Entschützung von der Fluorenylmethoxycarbonylgruppe wurde in 20% Piperidin in N,N-Dimethylformamid ausgeführt. Die Abspaltung der fertigen, teilgeschützten Peptide vom Träger(harz) erfolgte in einer 1:1 Mischung aus Essigsäure und Dichloromethan.
Im Falle Cysteinhältiger Peptide erfolgte die Seitenkettenentschützung nach der Abspaltung vom Träger(harz) in 95% Trifluoroessigsäure, worauf die Zyklisierung durch Oxidation der terminalen Cysteinreste mittels Luftzufuhr zum rohen, linearen Peptid bei einem basischen pH (8,5) innerhalb von 90 Stunden durchgeführt wurde. Das rohe, zyklische Peptid wurde mittels Reversed Phase Medium Pressure Flüssigkeitschromatographie ((RP-MPLC) über eine RP-C18 Silicagelsäule mit einem 5%-40 Acetronitril/Wasser Gradienten. Schließlich wurde das Trifluoroacetat-Ion über einer Lewatit MP64 Säule (Acetatform) durch Acetat ersetzt. Nach einer Waschung mit Wasser wurde das gereinigte Acetatsalz lyophilisiert und als weiße bis leicht gefärbtes Pulver erhalten.

Im Falle Cysteinfreier Peptide wurde der Zyklisierungsschritt am teilgeschützten linearen Peptid nach der Abspaltung vom Träger(harz) ausgeführt. Nach der selektiven Zyklisierung des Cysteinfreien Peptids erfolgte Seitenkettenentschützung mit Trifluoroessigsäure, gefolgt von präparativer RP-MPLC Chromatographie, Ersatz des Trifluoroacetat-Ions durch Acetat und Lyophilisierung der Acetatform, wie für die Cysteinhältigen Peptide.

Anschließend wurden die zyklischen Proteine SEQ ID 1 bis SEQ ID 6 mittels Reverser HPLC auf Reinheit und Masse untersucht.
Die Reinheit des zyklischen Proteins SEQ ID 1 war 96.3%. m/z (ESI) 1924.2 (M++1).
Die Reinheit des zyklischen Proteins SEQ ID 2 war 96.3%. m/z (ESI) 1924.2 (M++1).
Die Reinheit des zyklischen Proteins SEQ ID 3 war 98.8%. m/z (ESI) 1888.2 (M++1).
Die Reinheit des zyklischen Proteins SEQ ID 4 war 97.4%. m/z (ESI) 1873.4 (M++1).
Die Reinheit des zyklischen Proteins SEQ ID 5 war 99%. m/z (MALDI-TOF) 1901.6 (M++1).
Die Reinheit des zyklischen Proteins SEQ ID 6 war 99%. m/z (MALDI-TOF) 1902.7 (M++1).
Die Reinheit des zyklischen Proteins SEQ ID 7 war 95%. m/z (MALDI-TOF) 1778.02 (M++1).

Die Verbindung mit der Aminosäuresequenz SEQ ID NO:7
*Cyclo*(CGQREAPAGAAAKPWYC)
worin eine Disulfidbrücke zwischen den beiden endständigen Cysteinresten ausgebildet ist, hat sich als biologisch inaktiv erwiesen und wurde zu Vergleichszwecken genutzt.

### Beispiel 2

### Elektrophysiologische Untersuchungen des Amilorid-sensitiven Natrium Ionen Kanal (ENaC)

Makroskopische Natrium-Ionen Ströme wurden von menschlichen Lungenepithelzellen A549 in der "whole cell" Konfiguration mittels "Patch-clamp" Technik (Hamill et al, Pflugers Arch. 1981, 391(2):85-100., 1981) abgeleitet. Für die Stromableitungen in der "whole cell" Konfiguration wurden folgende Bad- und Elektrodenlösungen verwendet: Badlösung: 135 mM Natrium Methansulfonat, 10 mM NaCl, 2.7 mM KCl, 1.8 mM CaCl₂, 2 mM MgCl₂, 5.5 mM Glucose, und 10 mM HEPES, pH 7.4.

Elektrodenlösung: 120 mM Kaliummethansulfonat, 15 mM KCl, 6 mM NaCl, 1 mM Mg₂ATP, 2 mM Na₃ATP, 10 mM HEPES, and 0.5 mM EGTA (pH 7.2).

Deckgläser mit den darauf kultivierten Zellen wurden in ein 1 ml fassendes Versuchsbad transferiert, auf dem Mikroskoptisch (Axiovert 100, 400-fache Vergrößerung) fixiert und die Zellen mit der oben beschriebenen Badlösung superfundiert. Sodann wurde von einer geeigneten Zelle (welche am Deckglas haftet) der Strom abgeleitet. Dazu wurde eine mit einer Elektrolytlösung gefüllte Mikroelektrode (Glaskapillare mit einer definierten, Hitzepolierten Spitzenöffnung von ca. 1-3 µm (entspricht einem Widerstand der Elektrodenspitze von 3-5 MΩ) auf die Zelle aufgesetzt und die Membran angesaugt, sodass ein "Gigaohm-Seal" zwischen Membran und Elektrode gebildet wurde, um den Leckstrom zu minimieren. Bei der "whole cell"-Konfiguration wurde die Membran unter der Elektrodenspitze durchbrochen, damit der Strom, der durch alle Ionenkanäle der Zelle fließt, gemessen werden kann. Bei Erhalt eines "Gigaohm-Seals" wurde über einen Vorverstärker (CV-4 Headstage, Axon Instruments) und Verstärker (Axopatch 1D, Axon Instr.) ein definiertes Membranhaltepotential angelegt und der Strom, der dabei durch die Ionenkanäle fließt, gemessen.
Das Pulsprotokoll bestand aus einer Hyperpolarisation der Zellmembran auf -100 mV für 5 Sekunden und darauf folgender schrittweiser Depolarisation in 20 mV Schritten auf +100 mV.
Dieses Protokoll wurde vor (Kontrolle) und nach Zugabe der zyklischen Proteine durchgeführt. Die so erhaltenen Stromableitungen wurden gespeichert und mit Hilfe des Programms PCLAMP 6.0 analysiert. Dazu wurden die unter Anwesenheit von Amilorid erhaltenen Stromableitungen von den vorher registrierten Strömen subtrahiert, sodass der Amilorid-sensitive Natriumstrom durch die epithelialen Natriumkanäle ermittelt werden konnte.

Die Ergebnisse der Messungen sind in der Tabelle 1 zusammengefasst in der die Aktivität der zyklischen Proteine SEQ ID 1 bis SEQ ID 7 auf den zellularen Amilorid-sensitiven Natriumion Strom angegeben ist. Die Aktivität der einzelnen Peptide ist als EC₅₀ (in nM) angegeben. Die EC₅₀ ist die effektive Konzentration, bei der 50% von der maximalen Aktivität (d.h. maximale Erhöhung der Stromstärke, I) gemessen wird.

**Tabelle 1**

| **Zyklisches Protein** | **EC₅₀ (nM)** |
|---|---|
| | |
| SEQ ID NO:1 | 54 |
| SEQ ID NO:2 | 56 |
| SEQ ID NO:3 | 38 |
| SEQ ID NO:4 | 45 |
| SEQ ID NO:5 | 24 |
| SEQ ID NO:6 | 19 |
| SEQ ID NO:7 | keine Aktivität |

Die Aktivität der zyklischen Proteine SEQ ID 1 bis SEQ ID 6 in Abhängigkeit der Konzentration sind in der Abbildung 1 dargestellt. Die maximale Aktivität wurde mit 100% angegeben.

Die Untersuchungen, die in der Tabelle 1 und in Fig. 1 dargestellt sind, zeigen, dass die zyklischen Peptide mit den Aminosäuresequenzen SEQ ID NO:1 bis SEQ ID NO:6 biologisch aktiv sind, wohingegen das zyklische Peptid mit der Aminosäuresequenz SEQ ID NO:7, das strukturell eine gewisse Ähnlichkeit aufweist, nicht aktiv ist.

### Beispiel 3

### Herstellung des Lyophilisats

Die Entwicklung einer stabilen Lagerform der zyklischen Peptide der Formel I erfolgte im technischen Maßstab. Dazu wurden die zyklischen Peptide der SEQ ID NO:1 bis SEQ ID NO:6, sowie auch des zyklischen Peptids der SEQ ID NO:7 zu 0,1 mg/ml bis 100 mg/ml in reinem Wasser aufgelöst und zur Entfernung von Trübstoffen, Verunreinigungen und etwaigen Unsterilitäten durch Filter mit einer Porengröße von 0,2 µm filtriert.

Nach der Filtration wurde das in reinem Wasser gelöste zyklische Peptid in Glas-oder Plastikampullen portioniert und mittels Gefriertrocknung (Lyophilisierung) in ein stabiles Pulver überführt.

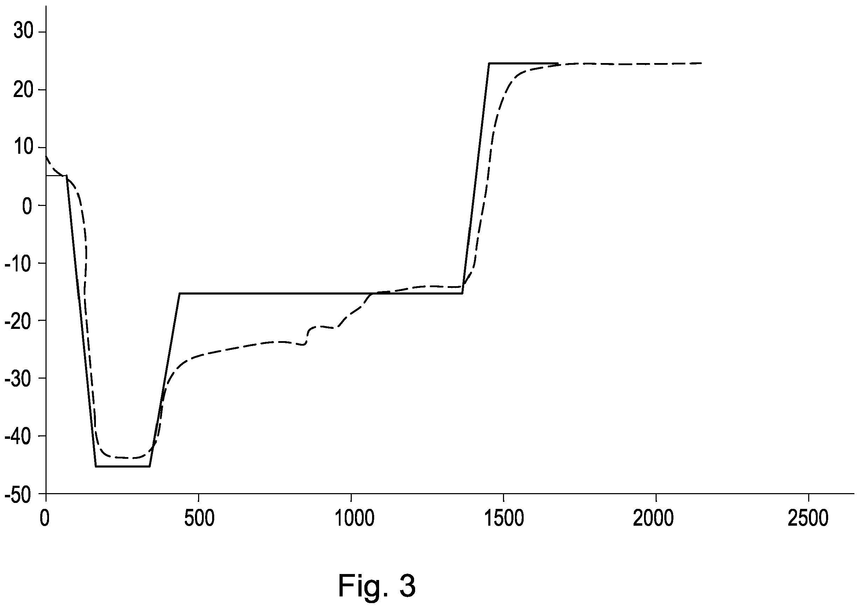

Dabei wurde der in Tabelle 2 bescriebenen Lyophilisationsparameter und der in Fig. 3 beschriebene Lyophilisationszyklus verwendet.

**Tabelle 2**

| **Schritt** | **Prozess** | **Fachtemperature (°C)** | **Haltezeit (min)** | **Temperaturveränderung (°C)während Haltezeit** | **Druck (mTorr)** |
|---|---|---|---|---|---|
| 1 | Laden | 5 | 60 | 5.0 | n/a |
| 2 | Gefrieren | -45 | 150 | 0.5 | n/a |
| 3 | Primäre Trocknung | -15 | 940 | 0.3 | 250 |
| 4 | Sekundäre Trocknung | 20 | 630 | 0.5 | 75 |
| 5 | Abschluß | Fläschchen unter 95% reiner Stickstoffatmosphäre verschlossen. | | | |

Als Ergebnis der Lyophilisierung wurde für die zyklischen Peptide mit den Aminosäuresequenzen SEQ ID NO:1 bis SEQ ID NO:6 jeweils ein weißes Pulver erhalten.

### Beispiel 4

### Stabilitätsuntersuchung des Lyophilisats aus Beispiel 2 nach Lagerung bei Raumtemperatur und im Kühlschrank

Die Stabilitätsuntersuchung des Lyophilisats der zyklischen Peptide mit den Aminosäuresequenzen SEQ ID NO:1 bis SEQ ID NO:6 erfolgte im technischen Maßstab. Das Lyophilisat wurde dazu für bis zu 24 Monate bei 2 bis 8°C und bis zu 6 Monate bei 25°C bei 60% relative Feuchtigkeit gelagert. Die Stabilität wurde zu verschiedenen Zeitpunkten während dieses Zeitraumes untersucht. Im Besonderen wurde das Aussehen, der Gehalt, sowie die Reinheit der zyklischen Peptide untersucht. Dafür wurden Labor-übliche analytische Verfahren verwendet, wie zum Beispiel die visuelle Inspektion und die Reverse HPLC.

Außerdem wurde nach einer Lagerung von 24 Monate bei 2-8°C die biologische Aktivität mittels Patch Clamp Experimente ermittelt. Dabei wurden makroskopische Ströme von A549 Zellen in der "whole cell" Konfiguration der "Patch-clamp" Technik abgeleitet, wie unter "Elektrophysiologische Untersuchungen des Amilorid-sensitiven Natrium Ionen Kanal (ENaC)" beschrieben.
In der Tabelle 3 sind die Ergebnisse der Stabilitätsuntersuchung des jeweiligen Lyophilisats der zyklischen Peptide mit den Aminosäuresequenzen SEQ ID NO:1 bis SEQ ID NO:6 zum Zeitpunkt T = 0 und nach 6 Monaten (T = 6M) bzw. nach 24 Monaten (T = 24M). zusammengefasst. Das Aussehen hat sich während des gesamten Zeitraumes nicht verändert. Der Gehalt an zyklischem Peptid, sowie die Reinheit unterlag nur geringfügen Schwankungen.

**Tabelle 3**

| | **Lagertemperatur 2-8°C** | | **Lagertemperatur 25°C / 60% rF** | |
|---|---|---|---|---|
| **Analytischer Parameter** | | | | |
| | **T=0** | **T=24M** | **T=0** | **T=6M** |
| **Aussehen** | | | | |
| SEQ ID 1 | weißes Pulver | | weißes Pulver | |
| SEQ ID 2 | weißes Pulver | | weißes Pulver | |
| SEQ ID 3 | weißes Pulver | | weißes Pulver | |
| SEQ ID 4 | weißes Pulver | | weißes Pulver | |
| SEQ ID 5 | weißes Pulver | | weißes Pulver | |
| SEQ ID 6 | weißes Pulver | | weißes Pulver | |
| | | | | |

| **Menge/Gehalt** | | | | |
|---|---|---|---|---|
| SEQ ID 1 | 100% | 99% | 100% | 99% |
| SEQ ID 2 | 100% | 99% | 100% | 99% |
| SEQ ID 3 | 100% | 99% | 100% | 99% |
| SEQ ID 4 | 100% | 99% | 100% | 99% |
| SEQ ID 5 | 100% | 99% | 100% | 99% |
| SEQ ID 6 | 100% | 99% | 100% | 99% |
| | | | | |

| **Reinheit** | | | | |
|---|---|---|---|---|
| SEQ ID 1 | 96% | 96% | 96% | 95% |
| SEQ ID 2 | 96% | 96% | 96% | 95% |
| SEQ ID 3 | 98% | 98% | 98% | 97% |
| SEQ ID 4 | 97% | 97% | 97% | 96% |
| SEQ ID 5 | 99% | 99% | 99% | 98% |
| SEQ ID 6 | 99% | 99% | 99% | 98% |

Das Lyophilisat jeweils eines der zyklischen Peptide mit den Aminosäuresequenzen SEQ ID NO:1 bis SEQ ID NO:6 ist demnach jeweils für bis zu 24 Monate bei 2 bis 8°C und bis zu 6 Monate bei 25°C/60% relative Feuchtigkeit stabil.

Die biologische Aktivitätsmessung mittels Patch Clamp Experimente ergab, daß ein Lyophilisat mit jeweils einem der zyklischen Peptide mit den Aminosäuresequenzen SEQ ID NO:1 bis SEQ ID NO:6 nach 24 Monaten Lagerung bei 2 bis 8°C immer noch voll aktiv war.

### Beispiel 5

### Herstellung einer wässrigen Lösung der zyklischen Peptide mit den Aminosäuresequenzen SEQ ID NO:1 bis SEQ ID NO:6 vor der Inhalation

Zur Vorbereitung zur Applikation der zyklischen Proteine mit den Aminosäuresequenzen SEQ ID NO:1 bis SEQ ID NO:6 wurden die stabilen weißen Pulver, die bei der Lyophilisierung gemäß dem Beispiel 2 erhalten wurden, jeweils in einem definierten Volumen an reinem Wasser zu einer Konzentration zwischen 0,1 mg pro ml und 100 mg pro ml aufgelöst. Die resultierenden Lösungen der zyklischen Proteine SEQ ID 1 bis SEQ ID 6 wurden dann in die Vorratsbehälter der Vernebler überführt. Durch Auflösen der zyklischen Proteine SEQ ID 1 bis SEQ ID 6 in Wasser ergaben sich klare Lösungen.

### Beispiel 6

### Untersuchung zur Stabilität der zyklischen Peptide mit den Aminosäuresequenzen SEQ ID NO:1 bis SEQ ID NO:6 in aufgelöster Zubereitung vor der Inhalation

Aus praktischen Gründen kann eine wässrige Lösung eines zyklischen Peptids mit einer der Aminosäuresequenzen SEQ ID NO:1 bis SEQ ID NO:6 nicht immer unmittelbar nach ihrer Herstellung zur Inhalation verwendet werden. Daher wurde exemplarisch die Stabilität einer wässrigen Lösung untersucht. Die fertig zu verwendende Lösung wurde dafür entweder in einer laborüblichen Spritze bei 2 bis 8°C für 7 Tage oder in der Kammer eines Verneblers bei 25°C für 24 Stunden aufbewahrt. Im Besonderen wurde das Aussehen, der Gehalt an zyklischem Protein SEQ ID 1 sowie dessen Reinheit untersucht. Die dafür angewandten Methoden waren laborübliche analytische Verfahren, wie zum Beispiele die visuelle Inspektion, sowie die Analyse mittels reverser HPLC.

Die Ergebnisse der Stabilitätsuntersuchung einer wässrigen Lösung des zyklischen Peptids mit der Aminosäuresequenz SEQ ID NO:1sind in der Tabelle 4 zusammengefasst. Das Aussehen hat sich während des gesamten Zeitraumes nicht verändert. Der Gehalt an zyklischem Peptid, sowie die Reinheit unterlag nur geringfügigen Schwankungen.

**Tabelle 4**

| **Parameter** | **Labor Spritze Temperatur 2bis 8°C** | | **Vorratskamer eines Verneblers, Temperatur 25°C** | |
|---|---|---|---|---|
| | **T=0** | **T=7 Tage** | **T=0** | **T=24 Stunden** |
| Aussehen | klare Lösung | | klare Lösung | |
| Menge/Gehalt | 25 mg/ml | | 25 mg/ml | |
| Reinheit | 96.3% | 96.2% | 96.6% | 96.5% |

Eine wässrige Lösung eines zyklischen Peptids mit der Aminosäuresequenz SEQ ID NO:1 bis SEQ ID NO:6 zur Inhalation ist demnach in einer laborüblichen Spritze bei 2 bis 8°C für 7 Tage oder in der Kammer eines Verneblers bei 25°C für mindestens 24 Stunden stabil.

### Beispiel 7

### Untersuchung zur Stabilität der zyklischen Peptide mit den Aminosäuresequenzen SEQ ID NO:1 bis SEQ ID NO:6 in aufgelöster Zubereitung während der Umwandlung in ein Aerosol

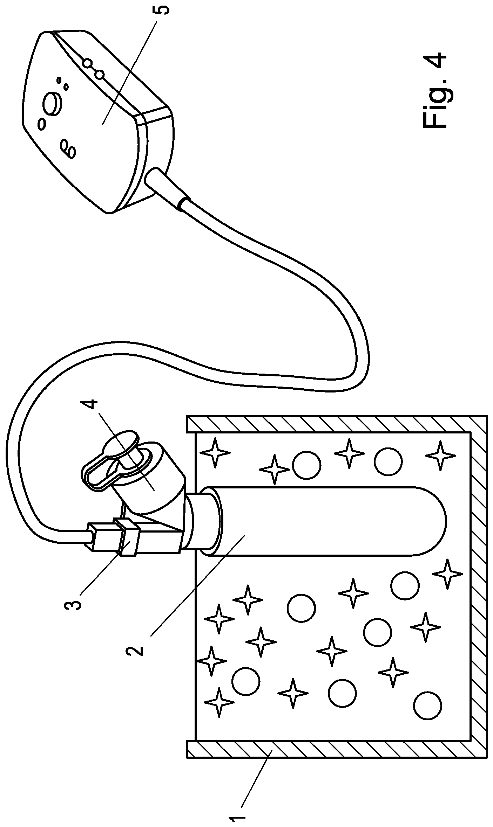

Da Proteine und Peptide mitunter sehr instabil sein können wurde untersucht, ob die zyklischen Peptide mit den Aminosäuresequenzen SEQ ID NO:1 bis SEQ ID NO:6 in aufgelöster Zubereitung während der Umwandlung in ein Aerosol stabil bleiben. Dazu wurden die zyklischen Peptide wie in Beispiel 4 beschrieben, in Wasser aufgelöst. Nach Einfüllen in den Vorratsbehälter eines Verneblers wurde die wässrige Lösung der zyklischen Proteine in ein Aerosol umgewandelt. Hierzu wurde eine "Mesh-Typ" Vernebler (Nebuliser) verwendet. Das aus dem Vernebler austretende Aerosol wurde dann in einer Kühlfalle, wie in Fig. 4 dargestellt, aufgefangen. Die biologische Aktivität des aufgefangenen Aerosols wurde mittels Patch Clamp Methode ermittelt. Dabei wurden makroskopische Ströme von A549 Zellen in der "whole cell" Konfiguration der "Patch-clamp" Technik abgeleitet, wie unter "Elektrophysiologische Untersuchungen des Amilorid-sensitiven Natrium Ionen Kanal (ENaC)" beschrieben.
Die chemische Stabilität des kondensierten Aerosols wurde mittels reverser HPLC/MS ermittelt.

Es konnte exemplarisch gezeigt werden, daß das zyklische Peptid mit der Aminsäuresequenz SEQ ID NO:1 in aufgelöster Zubereitung während der Umwandlung in ein Aerosol seine biologische sowie chemische Stabilität beibehält.

### Beispiel 8

### Physiko-chemische Charakterisierung des Aerosols der zyklischen Peptide mit den Aminosäuresequenzen SEQ ID NO:1 bis SEQ ID NO:6

Mit Hilfe eines Verneblers, welche wässrige Lösungen in ein Aerosol umwandelt, können auch die wässrigen Lösungen der zyklischen Peptide mit den Aminosäuresequenzen SEQ ID NO:1 bis SEQ ID NO:6 in Aerosole umgewandelt werden. Solche Aerosole können hinsichtlich der mittleren Tröpfchengröße sowie der Größenverteilung der Aerosoltröpfchen charakterisiert werden. Dazu werden 2 übliche Methoden verwendet, die auch in den Arzneibüchern (Pharmacopoeia) beschrieben sind. Die eine analytische Methode verwendet den Kaskadenimpaktor in dessen aktueller Bauart, den "Next Generation Impaktor". Dabei wird das Aerosol durch eine Reihe von Siebplatten geleitet, wobei der Durchmesser der Löcher mit jeder Platte kleiner wird und die Anzahl der Löcher zunimmt. Bei der anderen analytischen Methode, die Laser-Diffraktion Messung, werden die Tröpfchengrößen mittels Laser bestimmt. Die 2 wichtigen Kenngrößen, die bei diesen Messungen ermittelt werden ist einerseits der Median des Durchmessers aller Tröpfchen, und andererseits die Menge an Tröpfchen, die einen Durchmesser von ≤ 5 µm haben. In der Literatur wird dieser Durchmesser als Grenze beschrieben, unter der eingeatmete Aerosolpartikel auch tatsächlich in die Lunge gelangen.

Es ist weitläufig bekannt, daß in der Praxis von einem generierten Aerosol dem Patienten nur ein Teil zur Verfügung steht. Um also die Menge an Aerosol zu ermitteln, die einem Anwender zur Verfügung steht, wurden Versuche mit einem Atemsimulator durchgeführt. Zur Untersuchung der Partikelgröße und zur Durchführung der Atemsimulation wurde exemplarisch ein Aerosol aus einer wässrigen Lösung des zyklischen Peptids mit der Aminosäuresequenz SEQ ID NO:1 verwendet. Zur Erzeugung von Aerosol wurden verschiedene Vernebler Typen verwendet. Die Vernebler A und B waren sogenannte "Mesh-Typ Vernebler".

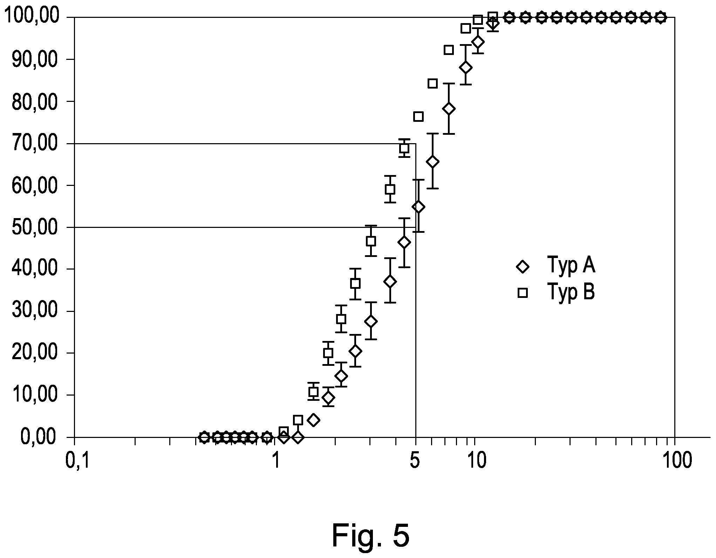

Der Anteil an Tröpfchen mit einem Durchmesser von ≤ 5 µm im Aerosol betrug bei allen Verneblern mindestens 50%., siehe Tabelle 5 und Fig. 5., worin Eigenschaften des Aerosols des zyklischen Peptids mit der Aminosäuresequenz SEQ ID NO:1 generiert von 3 verschiedenen Verneblern angegeben und gezeigt sind.

**Tabelle 5**

| **Vernebler** | **Median Partikeldurchmesser** | **Menge an Partikels mit Ø ≤ 5 µm** |
|---|---|---|
| Typ A | 4.7 µm | 50% |
| Typ B | 3.3 µm | 70% |
| Typ C | 3.7 µm | 65% |

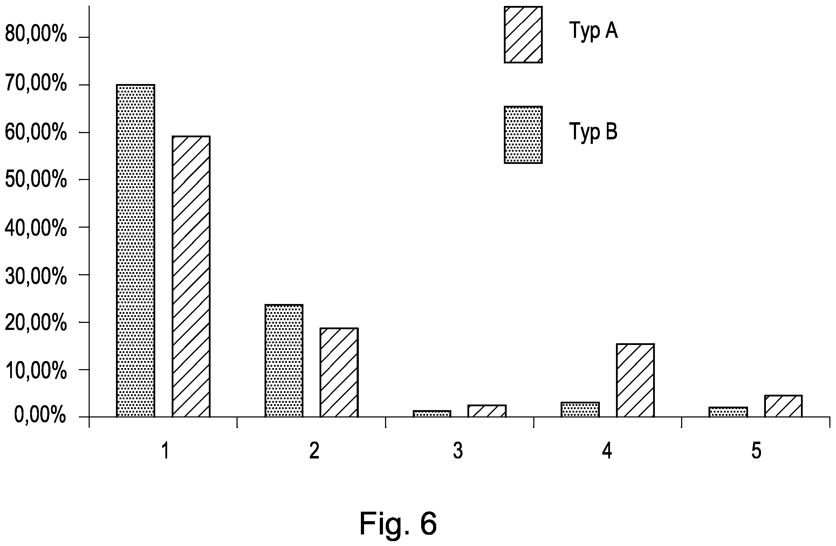

Weiters konnte nachgewiesen werden, dass der überwiegende Anteil der durch die Vernebler erzeugten Aerosole einer wässrigen Lösung des zyklischen Peptidds mit der Aminsäuresequenz SEQ ID NO:1 für die Inhalation zur Verfügung steht, wie in der Fig. 6 gezeigt, dargestellt durch den quantitativen Nachweis des zyklischen Proteins am "Inhalationsfilter". Die nicht zu Verfügung stehende Menge an Aerosol ist deutlich geringer (Fig. 6).

### Beispiel 9

### Nachweis der zyklischen Peptide mit den Aminosäuresequenzen SEQ ID NO:1 bis SEQ ID NO:6 im Blut nach parenteraler Verabreichung

Der exemplarische Nachweis der zyklischen Peptide mit den Aminosäuresequenzen SEQ ID NO:1 bis SEQ ID NO:6 nach parenteraler Verabreichung ins Blut erfolgte im Hund und in der Ratte. Dazu wurde exemplarisch eine wässrige Lösung des zyklischen Proteins mit der Aminosäuresequenz SEQ ID NO:1 als Bolus (25 mg/kg Körpergewicht) intravenös in die Versuchstiere appliziert. Unmittelbar nach Abschluss der intravenösen Applikation wurde Blut entnommen und mittels laborüblicher reverser HPLC/MS die Konzentration des zyklischen Proteins mit der Aminosäuresequenz SEQ ID NO:1 bestimmt. Die Ergebnisse sind in Tabelle 6 dargestellt, in der die Plasmakonzentration des zyklischen Proteins mit der Aminosäuresequenz SEQ ID NO:1 nach intraveöser Applikation angegeben ist.

**Tabelle 6**

| | Ratte | Hund |
|---|---|---|
| Plasmakonzentration (µg/ml) | 28 | 42 |

### Beispiel 10

### Nachweis der zyklischen Peptide mit den Aminosäuresequenzen SEQ ID NO:1 bis SEQ ID NO:6 im Lungengewebe nach Inhalation als Aerosol

Der Nachweis eines der zyklischen Peptide mit den Aminosäuresequenzen SEQ ID NO:1 bis SEQ ID NO:6 kann im Lungengewebe von Ratten erfolgen. Dazu wurde exemplarisch aus einer wässrigen Lösung des zyklischen Peptids mit der Aminosäuresequenzen SEQ ID NO:1 mit einem Vernebler ein Aerosol erzeugt. Das Aerosol wurde durch das Versuchstier eingeatmet (72 mg/kg Körpergewicht). Nach Abschluß der Inhalation vom Aerosol wurde sowohl das Lungengewebe als auch das Blut des Versuchstiers untersucht. Mittels reverser HPLC/MS wurde die Konzentration des zyklischen Peptids mit der Aminosäuresequenz SEQ ID NO:1 bestimmt.

Exemplarisch konnte nach einer Inhalation von insgesamt 72 mg/kg Körpergewicht das zyklische Peptid mit der Aminosäuresequenz SEQ ID NO:1 im Lungengewebe mit einer Konzentration von 1,2 µg/g Lungengewebe nachgewiesen werden. Im Unterschied dazu konnte bis zu einer Detektionsgrenze von 0,1 µg/ml das zyklische Peptid mit der Aminosäuresequenz SEQ ID NO:1 im Blut nicht nachgewiesen werden.

### Beispiel 11

### Effekt der Peptide mit den Aminsosäuresequenzen SEQ ID NO:1 bis SEQ ID NO:7 auf deglykosilierte Zelloberflächen

In Ganzzellenexperimenten wurden A549 Zellen mit dem Enzym "PNGase F" (Peptide-*N*⁴-(*N*-acetyl-*β*-D-glucosaminyl)asparagine Amidase F), 100 units für 1 bis 5 Minuten, unmittelbar vor den Oatch Clamp Messungen inkubiert und die Deckgläser mit den kultivierten Zellen wurden mit externer Lösung gespült, bevor sie in die Kammer des 1 mL Baders transferiert wurden.

Nach Kontroll After Kontrollaufzeichnungen wurden 240 nM der Peptide mit der Aminosäuresequenz SEQ ID NO:1 bis SEQ ID NO:7 zu der Badlösung zugegeben. Der Gesamte Zellstrom wurde aufgezeichnet bei Eh = -100 mV von Zellen ohne jegliche Vorbehandlung unter kontrollierten Bedingungen und nach der Zugabe eines der Peptide mit derAminosäuresequenz SEQ ID NO:1 bis SEQ ID NO:7, als auch mit Vorbehandlung mit PNGase F. Die Resultate der Deglykoslieirungsexperimente unter Verwendung des Patch Clamp Assays im Ganzzellenmodus zeigt die Tabelle 7, worin der Effekt der Deglykosilierung von A549 Zellen auf die Aktivierung des Natriumionenstroms durch die Peptide mit den Aminosäuresequenzen SEQ ID NO:1 bis SEQ ID NO:7 angegeben ist. Ganzzellenströme wurden aufgezeichnet bei Eh = -100 mV. Die Konzentration der Peptide mit den Aminosäuresequenzen SEQ ID NO:1 bis SEQ ID NO:7 in der Badlösung war 240 nM.

**Tabelle 7**

| **Kontrolle/Peptid** | **Vorbehandlung mit PNGase F** | **Keine Vorbehandlung mit PNGase F** |
|---|---|---|
| Kontrolle | 25.4 pA (n = 16) | |
| SEQ ID NO : 1 | 19.6 pA (n = 3) | 1073.3 ± 15.1 pA (n = 10) |
| SEQ ID NO :2 | 21.3 pA (n = 3) | |
| SEQ ID NO :3 | 20.6 pA (n = 3) | |
| SEQ ID NO :4 | 22.5 pA (n = 3) | |
| SEQ ID NO :5 | 22.4 pA (n = 3) | |
| SEQ ID NO :6 | 19.9 pA (n = 3) | |
| SEQ ID NO :7 | Keine Aktivität | Keine Aktivität |

Die Zellen mit PNGase F (Behandling) vor dem Patch Clamp Assay bauten die Fähigkeit der Peptide mit den Aminosäuresequenzen SEQ ID NO:1 bis SEQ ID NO:6 den Natriumstrom zu erhöhen, ab. Unter Kontrollbedingungen ohne Zugabe von Peptid in die Badlösung und bei einem Holding Potential von -100 mV, war der Natriumionenstrom 25.4 pA, sowohl in unbehandelten Zellen, als auch in Zellen, die mit PNGase F vorbehandelt waren. In unbehandleten Zellen resultierte die Zugabe von Peptiden mit der Aminosäuresequenz SEQ ID NO:1 bis SEQ ID NO:6 (Endkonzentration 240 nM) zu der Badlösung bei einem Holding Potential von -100 mV in einem merklichen Natriumionenstrom von mehr als 1.000 pA. Ein Peptid mit der Aminosäuresequenz SEQ ID NO:7 zeigte dagegen keine Aktivität.

### Beispiel 12

### Lungentransplantation Experimente mit Schweinen

Gehirntote Schweine wurden in Rückenposition gebracht und eine Längssternotomie wurde durchgeführt. Der Herzbeutel und beide Pleuralhöhlen wurden geöffnet. Die unter und obere Hohlvene wurden umschlossen. Ein Zuflusskatheter wurde in die Lungenarterie eingesetzt durch eine Raffnaht im rechten ventrikulären Ausflusstrakt. Ein Zuflussveschluß wurde erhalten durch Verbinden der untern und obern Hohlvene, Ausflussverschluß durch Abklammern der Aorta. Die Lungen wurden dann geschützt durch eine vorbeugende Spülung mit kalter, isotonischer Konservierungslösung (50 ml per kg Körpergewicht des Schweins, enthaltend Kaliumionen, Natriumionen, Magnesiumionen, Calciumionen, Chloridionen, Dextran, Glucose, Pufferionen) durch den Zuflusskatheter. Einschnitt des linken Herzrohrs ergibt Ausfluss. Die Lungen wurden während dieser Zeitspanne mit 50% Sauerstoff belüftet, und Eismatsch wurde in beide Pleuralhöhlen und und ins Mediastinum gegeben.

Die Explantationstechnik war als Ganzes die Entfernung mit Herz und Speiseröhre gemäß der folgenden Schritte
a) Dissektion der weichen Gewebebrücken zur Thoraxhöhle an beiden Seiten der Trachea.
b) Transsektion der beiden pulmonalen Ligamente (sehr tief, schwierige Einwirkung), dann vom VCI, der niedrigen thorakalen absteigenden Aorta, beziehungsweise der Speiseröhre.
c) Blunt-Abtrennung von restlichen mediastinalen Anhaftungen.
d) Komplette Aufblähung der Spenderlunge vor dem trachealen Abschluss mit einer Klammer.

Nach Explantation wurden die Lungen in Gaze gewickelt, in einen Eisbeutel, der mit Niedrig-Kalium-Dextran-extracellulärer-Lösung gefüllt war gelegt, und bei 4°C für 18 bis 24 Stunden aufbewahrt.

Für die ex-vivo Lungenkonditionierung wurde die EVLP-Technik (extravascular lung perfusion) verwendet. In der EVLP-Technik werden Spenderlungen in einen Kreislauf gesetzt, zusammengesetz aus einer Pumpe, Belüfter und Filtern. In der EVLP-Technik kann die Temperatur auf bis zu 37°C erhöht werden. In der EVLP wird ein Belüfter verwendet,um Sauerstoff zu den Lungen zu liefern. Die Pumpe wird verwendet, um die Lungen mit einer extrazellulären Lösung, die humanes Albumin und Nährstoffe enthält, zu durchströmen. Während der EVLP kann die Lungenfunktion regelmäßig betreffend Schlüsselindikatoren bewertet werden.

Für die experimentellen Schweinelungen-Transplantationsexperimente, wurde der EVLP Kreislauf mit 2.0 Liter menschlicher Albuminlösung geprimt. Diese extracelluläre Lösung hatte einen optimalen kolloidalen osmotischen Druck. Nachdem der Kreislauf entlüftet war, wurde der Prime bei 20°C zirkuliert, bis er mit den Lungen verbunden war. Heparin, Cefuroxim, Methylprednisolon wurden dem Perfusat beigegeben.

Die Aufbereitung der Spenderlunge des Schweins startete mit dem Annähen einer trichterförmigen Röhre aus Silastic mit einem drucküberwachenden Katheter, eingebaut in die linke atrialle Luftmanschette (LA), um die LA offen zu splinten und einen geschlossenen Perfusionskreislauf aurechtzuerhalten. Diese Röhre wurde abgesichert anastomisiert an die LA Luftmanschette unter Verwendung eines laufenden 5-0 Monofilament-Garns um eine zuverlässige und effektive Ausfluss Drainage zu erreichen. Der gleiche Kanülenetyp wurde verwendet für die Durchbohrung der Lungenarterie (PA), zurechtgemacht, wie erforderlich, um an die PA Größe anzugleichen . Eine retrograde Back-Table Spülung wurde ausgeführt unter Verwendung von 500 ml gepufferter, extracellulärer Lösung. Vor der Befestigung der Spenderlungen in den EVLP Kreislauf, wurde die Trachea geöffnet und direktes bronchiales Absaugen wurde ausgeführt, um die Atemwege zu reinigen. Eine endotracheale Röhre (Größe 8 mm I.D.) wurde in die Trachea eingeführt und mit einem Numbilicalen Band fest abgesichert. Danach wurden die Lungen transferiert in die EVLP Kreislaufeinheit. Zuerst, Vebindung der LA Kanüle mit dem Kreislauf und Initiierung eines langsamen, retrograden Flusses, um die PA Kanüle zu entlüften. Sobald die Entlüftung komplett war, wurde die PA Kanüle mit dem Kreislauf verbunden und anterograder Fluss von 150 ml/min wurde initiiert, mit dem Perfusat bei Raumtemperatur. In den nächsen 30 Minuten wurde die Temperatur des Perfusates dann schrittweise erhöht auf 37°C. Sobald die Temperatur 32-34°C erreicht hatte, wurde eine mechanische Belüftung der Spenderlungen der Schweine gestartet, wobei die Belüftungsrate und die Rate des Perfusatflussess schrittweise erhöht wurden..

Der Fluss des EVLP Gases befördert Sauerstoff zu den Lungen und liefert Kohlendioxid zum Zuflußperfusat (86% N₂, 6% O₂, 8% CO₂), mit Hilfe der Gasaustauschermembran wurde initiert (Start bei 0.5 L/min Gasfluss und Titrieren basierend auf das Zuflußperfusat pCO₂) den Zuflußperfusat(druck) pCO₂ zwischen 35-45 mm Hg aufrechtzuerhalten. In dem Moment, wo die Lunge voll expandiert war, wurde eine Einmaldosis AP301 (1 mg/kg in 5 ml Aqua), unter Verwendung eines einfachen flüssigen Standard Zerstäubersystems in die Spenderlunge des Schweins gegeben, ventiliert und durchströmt durch die EVLP Systemschaltung.

Während der EVLP Experimente wurde die Durchblutung konstant bestimmt. Die folgenden funktionellen Parameter wurden stündlich gemessen und aufgezeichnet:
- Fluss Pulmonararterie (PAF): L/min
- (mittlerer) Pulmonararteriendruck (PAP): mm Hg
- Linker Atrialdruck (LAP): mm Hg
- Pulmonarer Gefäßwiderstand (PVR= [PAP-LAP] x 80 / PAF): dynes/sec/cm-5
- mittlerer, Spitzen- und Plateaudruck des Atemwegs (mAwP, peak AwP, platAwP): cm H₂O
- dynamische Komplianz (Compliance) (mL/cm H₂O)
- Perfusat Gasanalyse-Zufluss (PA) und Ausfluss (PV) PO₂, PCO₂ und pH.

### Resultate

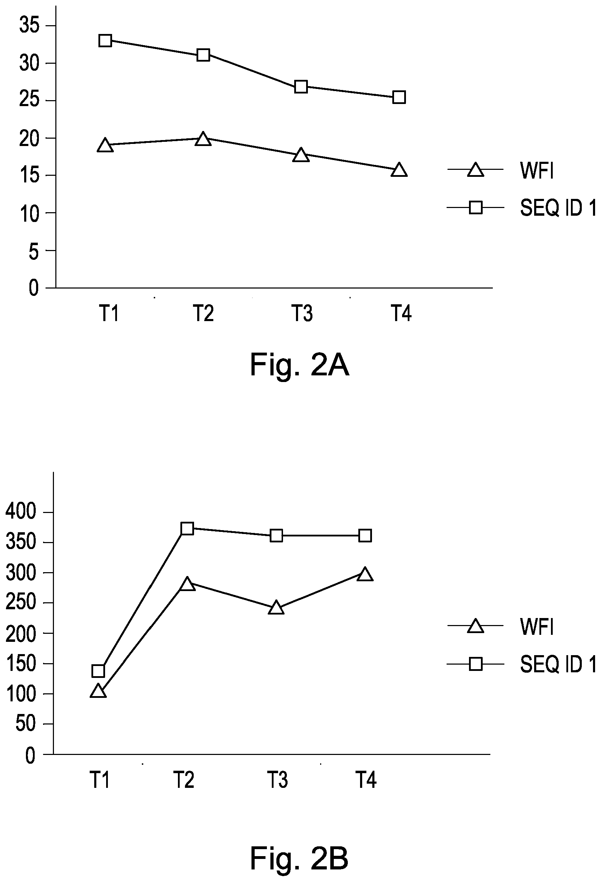

Dies Studie bewertet die Effekte eines Petids mit der Aminosäuresequenz SEQ ID NO:1 auf die Lungenfunktion in einem extrakorporalen System, das Lungentransplantation simuliert. Studienresultate zeigten, dass bei sich bei der Anwendung durch Einatmen beides, sowohl die dynamische Lungenkonformität, als auch die arteriovenöse pO₂ Differenz ΔpO₂ in Lungen, die mit einem Peptid mit der Aminosäuresequenz SEQ ID NO:1 behandelt wurden, verbessert wird, wie in Fig. 2A und Fig. 2B gezeigt ist.

Die Anwendung eines Peptids mit der Aminosäuresequenz SEQ ID NO:7 zeigte keine verbessernde Effekte auf die Lunge.

## Patentansprüche

1. Zyklisches Peptid der Formel
X₁-GQRETPEGAEAKPWY-X₂ I
worin
X₁ eine Aminsäure(sequenz) umfasst, mit 1 bis 4 Mitgliedern, umfassend natürliche oder unnatürlicheAminosäuren, und
X₂ eine natürliche Aminsäure umfasst, und worin
X₁ die N-terminale Aminosäure links in Position 1, und X₂ die C-terminale Aminosäure in der letzten, rechten Position enthält,
zur Verwendung als Arzneimittel zur Behandlung von pulmonaren Ödemen in der Form eines wässrigen Aerosols zur Inhalation ohne Zusatzstoffe und Stabilisatoren, erhältlich aus einem Lyophilisat des zyklischen Peptids der Formel I ohne Zusatzstoffe und/oder Stabilisatoren.

2. Zyklisches Peptid zur Verwendung nach Anspruch 1, in der X₁ in der Formel I ausgewählt ist aus der Aminsäure(sequenz) C (Cys), KSP (Lys-Ser-Pro), K (Lys), Ornithin, 4-Aminbuttersäure, β-Alanin,

3. Zyklisches Peptid zur Verwendung nach einem der Ansprüche 1 oder 2, in der X₂ in der Formel I ausgewählt ist aus der Gruppe C (Cys), D (Asp), G (Gly) und E (Glu),

4. Zyklisches Peptid zur Verwendung nach einem der Ansprüche 1 bis 3, worin eine zyklische Verbindung der Formel I ausgewählt ist aus einem der Peptide der Aminosäuresequenzen
- SEQ ID NO:1
*Cyclo*(CGQRETPEGAEAKPWYC)
worin zwischen den beiden terminalen Cysteinresten eine Disulfidbrücke ausgebildet ist;
- SEQ ID NO:2
*Cyclo*(KSPGQRETPEGAEAKPWYE) worin eine Amidbindung ausgebildet ist zwischen der Aminogruppe, die an das ε-Kohlenstoffatom des N-terminalen Lysinrestes gebunden ist und der Seitenketten-Carboxylgruppe, die an das γ-Kohlenstoffatem des C-terminalen Glutaminsäurerestes gebunden ist;
- SEQ ID NO:3
*Cyclo*(KGQRETPEGAEAKPWYG) worin eine Amidbindung ausgebildet ist zwischen der Aminogruppe, die an das ε-Kohlenstoffatom der Seitenkette des N-terminalen Lysinrestes gebunden ist mit der Carboxylgruppe des C-terminalen Glycinrestes;
- SEQ ID NO:4
*Cyclo*(Ornithin-GQRETPEGAEAKPWYG) worin eine Amidbindung ausgebildet ist zwischen der Aminogruppe, die an das δ-Kohlenstoffatom der Seitenkette des N-terminalen Ornithinrestes gebunden ist und der Carboxylgruppe des C-terminalen Glycinrestes;
- SEQ ID NO:5
Cyclo(4-Aminobuttersäure-GQRETPEGAEAKPWYD) worin eine Amidbindung ausgebildet ist zwischen der Aminogruppe des N-terminalen 4-Aminobuttersäurerestes und und der Seitenketten-Carboxylgruppe, die an das β-Kohlenstoffatom des C-terminalen Aspagraginsäurerestes gebunden ist; und
- SEQ ID NO:6,
Cyclo(β-alanine-GQRETPEGAEAKPWYE) worin eine Amidbindung ausgebildet ist zwischen der Aminogruppe des N-terminalen β-Alaninrestes (3-Aminopropansäurerest) und der Seitenketten-Carboxylgruppe, die an das γ- Kohlenstoffatom des C-terminalen Glutaminsäurerestes gebunden ist.

5. Zyklisches Peptid zur Verwendung nach einem der Ansprüche 1 bis 4, worin eine zyklische Verbindung der Formel I in der Form eines Salzes vorliegt.

6. Zyklisches Peptid zur Verwendung nach einem der Ansprüche 1 bis 5, worin die Partikelgröße des Aerosols, das zur Versprühung gelangt, einen Durchmesser von ≤ 5 µm aufweist.

7. Verfahren zur Herstellung eines wässrigen Aerosols umfassend ein zyklisches Peptid der Formel I wie in einem der Ansprüche 1 bis 6 definiert, wobei ein Lyophilisat des zyklischen Peptids der Formel I ohne Zusatzstoffe und Stabilisatoren in Wasser gelöst und ohne Zusatzstoffe und Stabilisatoren versprüht wird.

## Claims

1. Cyclic peptide of formula
X₁-GQRETPEGAEAKPWY-X₂ I,
wherein
X₁ comprises an amino acid (sequence), with 1 to 4 members, comprising natural and unnatural amino acids, and
X₂ comprises a natural amino acid, and wherein
X₁ comprises the N-terminal amino acid left in position 1, and X₂ the C-terminal amino acid in the ultimate, right position,
for use as a medicament for treating pulmonal edema,
in the form of an aqueous aerosol for inhalation without additives and stabilizers, obtained from a lyophilisate of the cyclic peptide of formula I without additives and/or stabilizers.

2. Cyclic peptide for use according to claim 1, wherein X₁ in formula I is selected from the amino acid (sequence) C (Cys), KSP (Lys-Ser-Pro), K (Lys), ornithine, 4-amino butyric acid, β-alanine.

3. Cyclic peptide for useaccording to any one of claims 1 or 2, wherein X₂ in formula I is selected from the group C (Cys), D (Asp), G (Gly) and E (Glu).

4. Cyclic peptide for useaccording to any one of claims 1 to 3, wherein a cyclic compound of formula I is selected from a peptide of the amino acid sequences
- SEQ ID NO:1
*Cyclo*(CGQRETPEGAEAKPWYC)
wherein a disulfide bridge is formed between both of the terminal cysteine residues;
- SEQ ID NO:2
*Cyclo*(KSPGQRETPEGAEAKPWYE)
wherein an amide bond is formed between the amine group which is attached to the ε-carbon atom of the N-terminal lysine residue and the side chain carboxy group, attached to the γ-carbon atom of the C-terminal glutaminic acid residue;
- SEQ ID NO:3
*Cyclo*(KGQRETPEGAEAKPWYG)
wherein an amide bond is formed between the amine group which is attached to the ε-carbon atom of the side chain of the N-terminal lysine residue with the carboxy group of the C-terminal glycine residue;
- SEQ ID NO:4
*Cyclo*(ornithine-GQRETPEGAEAKPWYG)
wherein an amide bond is formed between the amine group which is attached to the δ-carbon atom of the side chain of the N-terminal ornithin residue and the carboxy group of the C-terminal glycine residue;
- SEQ ID NO:5
*Cyclo*(4-amino butyric acid-GQRETPEGAEAKPWYD)
wherein an amide bond is formed between the amine group of the N-terminal 4-amino butyric acid residue and the side chain carboxy group which is attached to the β-carbon atom of the C-terminal asparaginic acid residue; and
- SEQ ID NO:6,
*Cyclo*(β-alanine-GQRETPEGAEAKPWYE)
wherein an amide bond is formed between the amine group of the N-terminal β-alanine residue (3-amino propanoic acid residue) and the side chain carboxy group, which is attached to the γ-carbon atom of the C-terminal glutaminic acid residue.

5. Cyclic peptide for useaccording to any one of claims 1 to 4, wherein a cyclic compound of formula I is in the form of a salt.

6. Cyclic peptide for use according to any one of claims 1 to 5, wherein the particle size in the aerosol which is going to be sprayed has a diameter of ≤ 5 µm.

7. Method of preparing an aqueous aerosol comprising a cyclic peptide of formula I as defined in one of claims 1 to 6, wherein a lyophyilisate of a cyclic peptide of formula I without additives and stabilizers is dissolved in water and sprayed without additives and stabilizers.

## Revendications

1. Peptide cyclique de formule
X₁-GQRETPEGAEAKPWY-X₂ I
dans lequel
X₁ comprend une (séquence) d'acides aminés avec un 1 à 4 composants, comprenant des acides aminés naturels ou artificiels et
X₂ comprend un acide aminé naturel et dans lequel
X₁ contient l'acide aminé N-terminal placé à gauche en position 1 et X₂ contient l'acide aminé C-terminal placé à droite en dernière position,
destiné à être utilisé comme médicament pour le traitement d'œdèmes pulmonaires sous la forme d'un aérosol aqueux à inhaler sans additifs ni stabilisateurs, pouvant être obtenu à partir d'un lyophilisat du peptide cyclique de formule 1 sans additifs et/ou stabilisateurs.

2. Peptide cyclique destiné à être utilisé selon la revendication 1, dans lequel X₁ dans la formule 1 est sélectionné parmi la (séquence) d'acides aminés C (Cys), KSP (Lys-Ser-Pro), K (Lys), ornithine, acide 4-aminobutyrique, β-alanine.

3. Peptide cyclique destiné à être utilisé selon une des revendications 1 ou 2, dans lequel X₂ dans la formule I est sélectionné parmi le groupe C (Cys), D (Asp), G (Gly) et E (Glu).

4. Peptide cyclique destiné à être utilisé selon une des revendications 1 à 3, dans lequel une liaison cyclique de la I est sélectionnée parmi un des peptides des séquences d'acides aminés suivantes :
- SEQ N° 1
Cyclo(CGQRETPEGAEAKPWYC)
dans laquelle un pont disulfure est formé entre les deux résidus de cystéine terminaux,
- SEQ N° 2
Cyclo(KSPGQRETPEGAEAKPWYE) dans laquelle une liaison amide est formée entre le groupe aminé qui est lié à l'atome de carbone ε du résidu de lysine N-terminal et le groupe carboxyle de la chaîne latérale qui est lié à l'atome de carbone γ du résidu d'acide glutamique C-terminal,
- SEQ N° 3
Cyclo(KGQRETPEGAEAKPWYG)
dans laquelle une liaison amide est formée entre le groupe aminé qui est lié à l'atome de carbone ε de la chaîne latérale du résidu de lysine N-terminal et le groupe carboxyle du résidu de glycine C-terminal,
- SEQ N° 4
Cyclo(ornithine-GQRETPEGAEAKPWYG) dans laquelle une liaison amide est formée entre le groupe aminé qui est lié à l'atome de carbone δ de la chaîne latérale du résidu d'ornithine N-terminal et le groupe carboxyle du résidu de glycine C-terminal,
- SEQ N° 5
Cyclo(acide 4-aminobutyrique-GQRETPEGAEAKPWYD) dans laquelle une liaison amide est formée entre le groupe aminé du résidu 4-aminobutyrique N-terminal et le groupe carboxyle de la chaîne latérale qui est lié à l'atome de carbone β du résidu d'acide asparagique C-terminal, et
- SEQ N° 6
Cyclo(β-alanine-GQRETPEGAEAKPWYE) dans laquelle une liaison amide est formée entre le groupe aminé du résidu de β-alanine (résidu d'acide 3-aminopropionique) et le groupe carboxyle de la chaîne latérale qui est lié à l'atome de carbone γ du résidu d'acide glutamique C-terminal.

5. Peptide cyclique destiné à être utilisé selon une des revendications 1 à 4, dans lequel une liaison cyclique de formule U se trouve sous forme de sel.

6. Peptide cyclique destiné à être utilisé selon une des revendications 1 à 5, dans lequel la taille des particules de l'aérosol qui parvient à la pulvérisation présente un diamètre ≤ 5 µm.

7. Procédé de fabrication d'un aérosol aqueux comprenant un peptide cyclique de formule I tel qu'il est défini dans une des revendications 1 à 6, dans lequel un lyophilisat du peptide cyclique de formule I est dissous dans l'eau sans additifs ni stabilisateurs et pulvérisé sans additifs ni stabilisateurs.
